# EUROPEAN PATENT APPLICATION

(11) **EP 3 909 594 A1**
(43) Date of publication of application: **17.11.2021**
(21) Application number: 20382405.7
(22) Date of filing: 14.05.2020
(51) Int. Cl.: A61K 35/28, A61K 39/395, A61K 31/727, A61K 35/32, A61K 31/573, A61K 35/51, A61K 31/711, A61K 9/00, A61P 19/00, A61P 37/02, A61P 31/14

(54) **MESENCHYMAL STROMAL CELLS FOR THE TREATMENT OF COVID-19 AND OTHER INFLAMMATORY, AUTOIMMUNE AND DEGENERATIVE DISEASES**

(71) Applicant: Soria Escoms, Bernat, 03540 Alicante (ES)
(72) Inventor: Soria Escoms, Bernat, 03540 Alicante (ES)
(74) Representative: Pons

(57) **Abstract**

The invention relates to the field of medical treatments, more specifically using mesenchymal stromal cells and extracellular vesicles in suspension to treat patients with inflammatory, autoimmune and degenerative diseases and their complications and more specifically for COVID-19. These diseases are currently accompanied by coagulation dysfunction, endothelial inflammation and macrophage activation. This "Off-the-self" New Pharmaceutical Products may be used either alone or in combination with other treatments such as defibrotide, anticoagulants, steroids, antiinterleukin 6, antiinterleukin 1, anti TNF alfa, cannabinoids and other therapeutic strategies to diminish or stop inflammation, autoimmunity and degenerative diseases, some of them such as COVID-19 without an effective treatment. Since there is no an effective vaccination for the virus SARS-CoV-2, this inventionrespond to an urgent need of new treatments for COVID-19 and other diseases. Although treatment with mesenchymal stromal cells of other origins than umbilical cord have been suggested for COVID-19 and some clinical trials already registered in the database ClinicalTrials.gov, no clinical data have been reported so far, even more, the exact conditions for this treatment to be safe and effective are described for the first time in this patent. The data presented in this patent show that intravenous administration of an "Off-the-self" Pharmaceutical Product consisting in: a) Mesenchymal stromal cells obtained either from adipose-tissues or from bone-marrow, b) Expanded in a culture media that may contain fetal bovine serum (FBS), human platelet lysate, growth factors or even better with a completely xeno-free and human component free media,) cryopreserved and thawed, d) Administered intravenously by an accredited medical person at a flux of 1-3 ml/min and e) in one or several doses of 0,75-1,5 million cells per kilogram. Also covered by this Patent are: a Pharmaceutical Product consisting is the extracellular vesicles and exosomes obtained from these cells. The pharmaceutical products described in this patent are original, not obvious and have an obvious commercial application.

## Description

### FIELD OF THE INVENTION

The invention relates to the field of medical treatments, more specifically using mesenchymal stromal cells and extracellular vesicles in suspension to treat patients with inflammatory, autoimmune and degenerative diseases and their complications and more specifically for COVID-19. These diseases are currently accompanied by coagulation dysfunction, endothelial inflammation and macrophage activation. This "Off-the-self" New Pharmaceutical Products may be used either alone or in combination with other treatments such as defibrotide, anticoagulants, steroids, antiinterleukin 6, antiinterleukin 1, anti TNF alfa, cannabinoids and other therapeutic strategies to diminish or stop inflammation, autoimmunity and degenerative diseases, some of them such as COVID-19 without an effective treatment. Since there is no an effective vaccination for the virus SARS-CoV-2, this invention respond to an urgent need of new treatments for COVID-19 and other diseases. Although treatment with mesenchymal stromal cells of other origins than umbilical cord have been suggested for COVID-19 and some clinical trials already registered in the database ClinicalTrials.gov, no clinical data have been reported so far, even more, the exact conditions for this treatment to be safe and effective are described for the first time in this patent. The data presented in this patent show that intravenous administration of an "Off-the-self" Pharmaceutical Product consisting in: a) Mesenchymal stromal cells obtained either from adipose-tissues or from bone-marrow, b) Expanded in a culture media that may contain fetal bovine serum (FBS), human platelet lysate, growth factors or even better with a completely xeno-free and human component free media,) cryopreserved and thawed, d) Administered intravenously by an accredited medical person at a flux of 1-3 ml/min and e) in one or several doses of 0,75-1,5 million cells per kilogram. Also covered by this Patent are: a Pharmaceutical Product consisting is the extracellular vesicles and exosomes obtained from these cells. The pharmaceutical products described in this patent are original, not obvious and have an obvious commercial application.

### BRIEF DESCRIPTION OF THE INVENTION

### SUMMARY OF THE INVENTION

We have developed a safe and effective treatment of patients of COVID-19 and other inflammatory, autoimmune and degenerative diseases by administrations of allogenic mesenchymal stromal cells either alone or in combination with other medicaments.

In **one aspect** mesenchymal stromal cells obtained from different sources of healthy donors such as bone marrow, adipose tissue, dental pulp, umbilical cord Wharton jelly and others are administered in one or more doses of 0,5 to 5 million of cells per kg by intravenous administration and simultaneous control of pulmonary artery pressure.

In a preferred embodiment cells are obtained and cultured in a media containing chemically defined media free of components of animal origin, such as Fetal Bovine Serum, or human origin, such as platelet lysate.

In a preferred embodiment the pharmaceutical product is an off-the-self Advanced Therapy Medicinal Product cryopreserved and administered after thawing with a well-defined procedure cleaning several times the Cellular Medicament using the excipient to be used in the administration.

In a **second aspect** the invention relates to a New Pharmaceutical Product consisting in a suspension of mesenchymal stromal cells administered intravenously at a rate of 1 to 3 mL/min by a trained and qualified person, e.g a registered trained medical doctor.

In a preferred embodiment the pharmaceutical product is an off-the-self Advanced Therapy Medicinal Product cryopreserved and administered after thawing with a well-defined procedure cleaning several times the Cellular Medicament using the excipient to be used in the administration.

In a **third aspect** the invention relates to a New Pharmaceutical Product consisting in a suspension of mesenchymal stromal cells administered locally, for example intramuscularly in the leg or arm of the patients by a trained and medically qualified person, e.g a registered and trained medical doctor or a trained and registered medical nurse. Intramuscular administration allow local activation of mesenchymal stromal cells by nearby cytokines.

In a preferred embodiment the pharmaceutical product is an off-the-self Advanced Therapy Medicinal Product cryopreserved and administered after thawing with a well-defined procedure cleaning several times the Cellular Medicament using the excipient to be used in the administration.

In a **fourth aspect** the invention relates to a New Pharmaceutical Product consisting in a suspension of mesenchymal stromal cells administered intraperitoneally, for example in the omentum of the patients by laparoscopy by a trained and medically qualified person, e.g a registered and trained medical doctor.

In a preferred embodiment the pharmaceutical product is an off-the-self Advanced Therapy Medicinal Product cryopreserved and administered after thawing with a well-defined procedure cleaning several times the Cellular Medicament using the excipient to be used in the administration.

In a **fifth aspect** of the invention the pharmaceutical product is a suspension of extracellular vesicles or exosomes obtained from mesenchymal stromal cells.

In a preferred embodiment of the invention, mesenchymal stromal cells were cultured in a chemically defined media free of products of animal origin, such as fetal bovine serum and free of products of human origin, such as human platelet lysate.

In another preferred embodiment of the invention Extracellular vesicles are administered in an aerosol composition.

In a **sixth aspect** of the invention, the media free of fetal bovine serum and human platelet lysate is enriched with 3 to 10 mM lithium chloride, 3 to 10 mM glucose. 0.5 to 10 micromolar nitric oxide donor, 50 to 200 nM hydrocortisone, plus recombinant growth factors and physiological concentration of nutrients.

In a **seventh aspect** of the invention, the pharmaceutical product is administered in combination with other medicinal products such as defibrotide, steroids, anticoagulants or monoclonal antibodies against interleukin-6, interleukin-1, TNF-alfa, cannabinoids or other anti-inflammatory therapies.

In a preferred embodiment of the invention, mesenchymal stromal cells were cultured in a chemically defined media free of products of animal origin, such as fetal bovine serum and free of products of human origin, such as human platelet lysate.

### BRIEF DESCRIPTION OF THE DRAWINGS

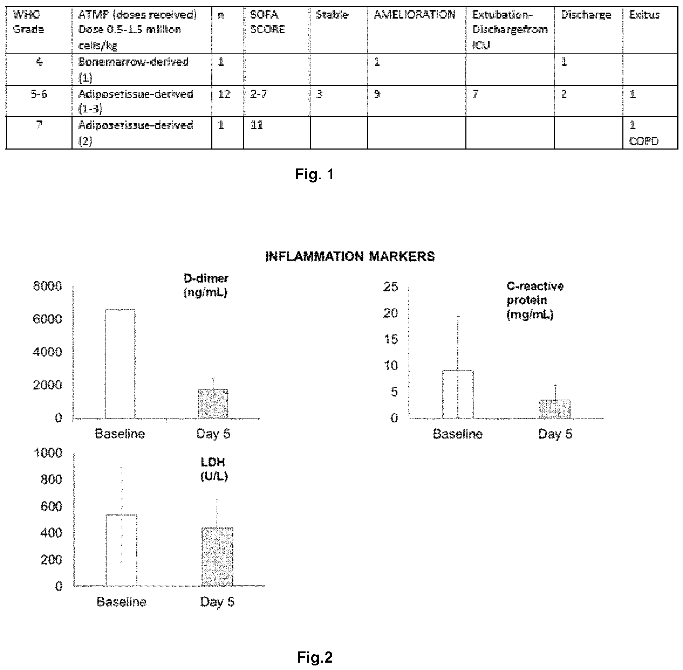

- Figure 1: Summary of the clinical outcomes in fourteen COVID-19 patients treated with safe and effective ATMPs. ATMP: Advanced Therapy Medicinal Product. WHO Grade: as described in World Health Organization. World Health Organization and R&D Blueprint strategy for COVID-19. Accessed May 2, 2020. https://www.who.int/teams/blueprint/covid-19. ICU: Intensive Care Unit. SOFA Score: Sequential organ failure assessment score (SOFA score), previously known as the sepsis-related organ failure assessment score (Singer, Mervyn; et al. (23 February 2016). The Third International Consensus Definitions for Sepsis and Septic Shock (Sepsis-3). JAMA. 315 (8): 801-10. doi:10.1001/jama.2016.0287)
- Figure 2: Treatment with the new Pharmaceutical Product inhibits the strong inflammatory response (D-dimer, C-reactive protein, LDH) in COVID-19 patients treated with 0.5 to 1,5 million cells /kg (n= 6)
- Figure 3: Treatment with the new Pharmaceutical Product ameliorates Lymphopenia of COVID-19 patients. Lymphocyte and lymphocyte subsets counts (Total lymphocytes, T- lymphocytes, B-lymphocytes, CD4+ and CD8+ lymphocytes; n=11).
- Figure 4: Intravenous administration of the New Pharmaceutical Products with simultaneous control of pulmonary artery pressure. ATMP: Advanced Therapy Medicinal Product, CV: cava vein, RA: right atria, RV: right ventricle, PA: pulmonary artery. The Swan-Granz catheter allows monitoring of pressure in the RA, RV and PA.
- Figure 5: Other routes of administration and biodistribution of ATMPs. A) Intraperitoneal administration route for NAFLD, Liver fibrosis, inflammatory bowel disease, Crohn's disease, Ulcerative colitis, type 1 diabetes and type 2 diabetes among others. B) Subcutaneous and intra-arterial administration routes for type 1 an type 2 diabetes. L: liver: P:Pancreas; C: Colon; O: Omentum; S: Spleen; MES: Mesenteric Artery.
- Figure 6: Doubling time and characterization of MSC (either bone marrow-derived in A or adipose tissue-derived in B). Comparison of Mesenchymal Stromal Cells expanded in conventional control media(C) or in xeno-free and human component-free chemically defined media (XF).

### COMPOSITION OF THE INVENTION

In the present application, the term "pharmaceutical product" makes reference to a mix of one or more active principles, with or without additives, whose physical characteristics are suitable for its dosage, preservation, administration and bioavailability.

A "solution" is a pharmaceutical product consisting of a liquid, transparent and homogeneous liquid obtained by dissolving the active principle or principles and the additives in water, and which is employed for external or internal use. In the case of injectable, ophthalmic and otic solutions, they must be sterile. The term "solution" includes dilutions.

A "suspension" is a pharmaceutical product consisting of a disperse system formed by two phases, which contain the active principle or principles and the additives. One of the phases, which is continuous or external, is generally a liquid or a semisolid, and a dispersed or internal phase is made by insoluble solids (active principles) which are dispersible in the external phase. In case it is injectable, it must be sterile.

An "aerosol" is a pressurized dosage form containing one or more therapeutic active ingredients which upon actuation emit a fine dispersion of liquid and/or solid materials in a gaseous medium

The term adipose tissue derived mesenchymal cells, bone marrow-derived mesenchymal stromal cells or umbilical cord-derived stromal cells, as used herein, refers to cells that originate from these tissues and are phenotypically characterized in that they are (i) negative for at least one, two, three, four, five, six, seven, eight, nine, ten or preferably all of the following markers CD3, CDI lb, CD14, CD19, CD31, CD34, CD45, CD62L, CD95L, CDI 17, and HLA-DR cell surface markers, and (ii) positive for at least one, two, three, four, five, six, seven, eight or preferably all of the following markers CD 13, CD29, CD44, CD49e, CD73, CD90, CD 105, CD 166, and HLA-ABC cell surface markers.

As used herein, the terms "treat", "treatment" and "treating" refer to the amelioration, cure or remedy of inflammatory disease, which results from the administration of the pharmaceutical composition provided by the present invention comprising a population of MSCs to a subject in need of said treatment.

Along the description and claims, the word "comprises" and variants thereof do not intend to exclude other technical features, supplements, components or steps. For persons skilled in the art, other objects, advantages and features of the invention will be understood in part from the description and in part from the practice of the invention. The following examples and drawings are provided by way of illustration and they are not meant to limit the present invention.

### Ethics approval and consent to participate

The studies were approved by the Ethical Committees of the respective hospitals and the National Agencies of Medicaments and Sanitary Products (AEMPS). Informed consent was obtained by patients when possible or their legal tutors.

### DETAILED DESCRIPTION OF THE INVENTION

This invention responds and gives a solution to the observation of why clinical trials with Advanced Therapies Medicinal Products and other Pharmaceutical Products result in heterogeneous and sometimes contradictory results. A pharmaceutical Product is currently formed by an Active Principle (Mesenchymal Stromal Cells) in a particular formulation (for example suspension of an "Off-the self" cryopreserved suspension of Mesenchymal Stromal Cells), thawed or refreshed before administration and administered with a strict protocol. This invention describes the method and components needed to reach better clinical outcomes. Advanced Therapies are "living medicaments", consequently, most of the heterogeneous results described may be explained by this new concept in Pharmacology. The advances reported here may be summarized following the definition of Cellular Medicament. In order for this Pharmaceutical Products to be considered an Advanced Therapies Medicinal Products a strict protocol has to be followed in which: 1 the properties, physiology and cell biology of the active principle in the formulation used) are taken in account; 2) the moment, dose and pattern of administration determined by the pathophysiology and pathogenesis of the process to be treated; 3) incorporates the pharmacodynamy and biodistribution of the Cellular Medicament and their interaction with other medicaments; and 4) increase the safety, feasibility and effectiveness by controlling the interaction of the Cellular Medicament with the body tissues, cells and fluids. Furthermore, safety, feasibility and efficacy of an Advanced Therapy Medicinal Product is also dependent of other factors such as: 5) Type of Cell used; 6) Culture media for the expansion of the cellular medicament, especially when the culture media contain or not products of animal origin such as fetal bovine serum or products of human origin such as platelet lysate; 7) Method of cryopreservation, thawing and resuspension and 8) the absolute need that the administration of the Pharmaceutical Product has to be done by a trained and accredited healthcare professional. Accomplishing all these conditions is compulsory for an ATMP to result better clinical outcomes and reinforces the value of this invention.

### Definition of Cellular Medicament ATMP

According to the European Committee for Advanced Therapies of the European Medicament Agency, the Food and Drug Administration and other international agencies, ATMPs are defined not only by the tissue source of the cells (i), but also for: the manufacture process (ii), the storage, cryopreservation, thawing and resuspension (iii) and the administration procedure (iv). As previously described, allogeneic mesenchymal stromal cells may be obtained either from bone marrow, adipose tissue, dental pulp, umbilical cord Wharton jelly and others; manufactured under conventional GMP conditions or with culture media free of components from animal origin or human origin.

### Inflammatory, autoimmune and degenerative diseases

The physiology of healing incorporates inflammation and regeneration, a not well known crossroad. A series of concomitant and interactive responses maintains tissue homeostasis. Inflammation underlies a wide variety of processes. The classic instigators of inflammation are infection and tissue injury, but other processes such as inmmmune disease, cancer, etc) constitute a range of adverse conditions that may induce inflammation, triggering the recruitment of leukocytes and plasma proteins to the affected tissue site. Then tissue stress or malfunction similarly induces an adaptive response, which is referred to here as **para-inflammation.** This response relies mainly on tissue-resident macrophages and is intermediate between the basal homeostatic state and a classic inflammatory response. Para-inflammation is probably responsible for the chronic inflammatory conditions that are associated with modern human diseases [1]. The variety of responses include acute inflammation process, chronic inflammation process, etc. For example. Inflammatory bowel disease, Crohn's disease, ablepsia disease, atopic dermatitis, erectile dysfunction, etc. Recent and anticipated advances in the field of immunology, and the increasing recognition of inflammation as an important component of neurodegeneration, are shaping our conceptualization of disease pathophysiology, and we explore the potential implications for improved healthcare provision to patients in the future [2]. Inflammation also accompanied degenerative diseases, neurodegenerative diseases (Alzheimer's disease, Parkinson's disease, amyotrophic lateral sclerosis, Parkinson-dementia), musculoskeletal disorders (Duchenne's muscular dystrophy), immunodiseases (multiple sclerosis, lupus) and cancer-related syndromes (multiple myeloma, leukemia...) Inflammation-based pathogenesis such as erythema nodosum leprosum, etc.

### COVID-19: An example of Inflammatory, Immune and Degenerative Disease

COVID-19 is a new disease. Whilst we are learning about it, several syndromes seem to parallel the clinical manifestations of COVID-19 cytokine storm: i) Graft-versus-Host Disease (GVHD) after a hematopoietic stem cell transplantation. li) Macrophage Activation Syndrome and iii) secondary Haemophagocytic LymphoHistiocytosis (sHLH) an under recognized hyperinflammatory syndrome triggered in adults by viral infections with fever, cytopenia and hyperferritinemia¹ that resembles that taking place in COVID-19 patients with poor prognosis.

### Critical situation of the Intensive Care Units

As the epidemic is progressing, the main problem facing the healthcare systems, as is the case worldwide, is the saturation of beds in the Intensive Care Units (ICU). There is an urgent need to treat patients suffering from COVID-19 respiratory distress to improve their clinical condition and allow these patients to be free from mechanical ventilation and discharged from ICUs. We propose a New Advanced Therapy Medicinal Product (ATMP) described in this invention consisting in intravenous administration of an "off-the-self" cryopreserved and thawed before administered consisting in mesenchymal stromal cells administered with astrict protocol in patients with different levels of respiratory distress, pulmonary and systemic inflammation and thrombotic problems caused by SARS CoV2. Our goal for high risk ICU patients (WHO Grade 5 to 7) is that they experiment a milder level of symptoms and leave the ICU in the shortest time, milder degrees (WHO Grade 3 and 4) should not enter in the ICUs and most probably be discharged. Figures 1-3 summarize the beneficial effect of this treatment.

One of the most striking facts of the physiopathology of pneumonia in COVID-19 disease [3] is the development of a massive inflammatory phase [4,5], with elevation of numerous acute phase reactants and cytokines (e.g. ferritin, C-reactive protein, fibrinogen, creatine kinase, LDH, or IL-6), leading to acute respiratory distress syndrome (ARDS) and macrophage activation syndrome (MAS)-like disease [6,7]. In addition, the presence of a progressive endothelial thrombo-inflammatory syndrome (with elevated D-dimers) not described in other viral infections adds differential features and aggravates the disease's prognosis [8-10]. With this treatment we demonstrate that our "off-the-self" product ameliorates the COVID-19 symptoms and signs.

Other data supporting this virally driven hyperinflammation could be the hypercoagulability with elevated plasma D-dimer, ferritin and IL-6 levels happening in these patients, being also by now the best predictors of fatality for this complication [11]. Under this hyperinflammatory, hypercoagulability and endothelial damage clinical setting, immunosuppression seems to be likely beneficial as it has been proven in recent controlled randomized clinical trials using proinflammatory cytokine blockade biologic drugs in COVID-19 patients [12,13,14]. Based on these data, open therapeutic options are already being considered such as low-dose steroids that should be used very carefully to avoid a potential exacerbation of lung injury, IL-6 (i.e. tocilizumab) and IL-1 (i.e. anakinra) receptor antagonists, or JAK antagonists. Other therapeutic options could be TNF antagonists or even IFN-*γ* antagonist; in fact, there are already clinical trials underway with the latter, not exactly on COVID-19, but on some hemophagocytic lymphohistiocytosis. However COVID-19 clinical outcomes also include hypercoagulability with thromboembolism and organ tissue damage. Mesenchymal stromal cells (MSCs) from different origins, cultured and expanded either with conventional GMP media containing fetal bovine serum or human platelet lysate or in chemically defined culture media with no components either from animal or human origin and administered with a specific protocol are key therapeutic weapons for COVID-19 and other diseases. So far, more than 20 clinical trials have been proposed to use mesenchymal stem cells for COVID19 patients, however there are not published data except for the paper of Leng et al [15] in which umbilical cord derived mesenchymal stem cells were used, but not the adipose tissue-derived or bone marrow-derived mesenchymal stromal cells as described in this Patent. Not to mention all the innovations described in the Examples.

In addition to coagulation dysfunction and vascular damage (vasculitis, Kawasaki syndrome), COVID-19 patients display a variety of signs of multiorgan damage. In principle, SARS-CoV-2 binds angiotensin converting enzyme 2 (ACE-2), thus enters and affects those cells that express ACE-2. In consequence, SARS-CoV-2 affects not only lungs but other tissues such as heart and vessels, kidney, brain, gastrointestinal tract and liver. Heart and Vessels: the mechanism by which SARS-CoV-2 attack heart and vessels are not well known. Results from Wuham scientists describe that 20 to 40% patients display symptoms of heart damage and arrhythmias. The fact the one third of patients in China and Europe have prothrombosis and coaguli together with high figures od D-dimer (a subproduct of blood coaguli). Kidney: this organ express high concentration of ACE2 receptors, subsequently one third of Wuham patients have kidney failure and urine proteins. Gastrointestinal Tract: some patients begin with diarrhea, vomiting, and faeci positive for SARS-CoV-2. Liver: approximately 50% of hospitalized patients have high enzyme levels. To be noted , drug metabolism may explain in part these signs. Eves and Nose mucosae: with conjunctivitis, etc. Brain: a minor percentage of patients (5-10%) had a brain inflammatory encephalitis with a strong symptomatic response. Earlier symptoms such as loss of smell and taste may reflect a damage in the extremely important brain stem reflexes and, even more, sudden death observe in hypoxemia patients without compensatory reflexes.

Are there basis for a New and Safe "Off-the-Self" Pharmaceutical Product with a wide mechanism of action that may benefit these patients?

### The rationale: Expected effects of Mesenchymal Stromal Cells (MSC).

### Advantages of MSC over other treatments

The active principle of this New Pharmaceutical Product have demonstrated anti-inflammatory [16] and fibrinolytic activity [17,18]. Furthermore, it has been shown that also promote tissues and cell regeneration. In addition, these cells are capable of regulating the polarization of proinflammatory macrophages of type M1 (classical activation) to those of type M2 (anti-inflammatory, alternatively activated) [19,20]. Then, MSC administration tends to unbalance the pro-inflammatory cytokines, immune cells and tissue damage towards an anti-inflammatory and regenerative microenvironment. Inthis sense MSCs have been widely used in cell-based therapy, from basic research to clinical trials. Safety and efficacy have been clearly documented in many clinical trials, especially in both systemic and local immune-mediated inflammatory diseases, such as GVHD, Crohn's complex fistula and type 2 diabetes complications [3,4,21]. MSCs play a positive role mainly in two ways, namely immunomodulatory effects and anti-inflammatory abilities, both linked to regeneration. MSCs, when activated, can secrete many types of cytokines by paracrine secretion or make direct interactions with immune cells. However, untrained healthcare professionals do not follow the administration protocol (which is part of the Pharmaceutical Product) and adverse or unexpected reaction may appear. Even more, expansion in human platelet lysate or in fetal bovine serum containing culture media do not protect for certain reactions.

Theoretically, MSCs may act as suppressors of the cytokine storm, specifically through IL-1 blockade. Recent data support that IL-1 receptor antagonist, a naturally occurring antagonist of IL-1α/IL-1β signaling pathways, has been attributed to the immunosuppressive effects of MSCs [5]. So, IL-1 blockade seems to activate MSCs toward anti-inflammatory phenotype able of releasing anti-inflammatory cytokines, of increasing Treg and of favoring polarization of M1 (pro-inflammatory) macrophages into M2 (anti-inflammatory), which could contribute to revascularization and regeneration of lung tissue [7]. This is why these cells have been proposed for use in pulmonary sepsis, chronic obstructive pulmonary disease (COPD) and cystic fibrosis.

On the one hand, in the current pandemic situation, the use of the intravenous route is the most appropriate for the current ICUs' setting. Additionally, MSCs of any origin injected intravenously are rapidly located in the pulmonary microcirculation network because the cells are larger than the diameter of the capillaries, based on previous data in pre-clinical animal models. However, the risk for a sudden increase in the arterial pressure needs to be monitored. In principle they will be captured by local macrophages, which subsequently stimulate mesenchymal cells to produce IL-10, indirectly providing a source of immunosuppressive cytokines [9].

### EXAMPLE 1: Treatment of COVID-19 Patients (BALMYS-19 Project) with adipose tissue-derived and bone marrow-derived mesenchymal stromal cells

We here demonstrate that a New ATMP ("Off-the-self" Pharmaceutical Product already used in COVID-19 patients for the first time and described in this patent) can be beneficial in COVID-19 patients (Grade 5-7) with severe lung inflammation and oxygen therapy (mechanical ventilation), not excluding their use in earlier stage of the disease.

Even more, when this product is used in Grade 4 COVID-19 patients it may be concluded that early treatment precludes pulmonary damage and sequelae.

Adipose-derived mesenchymal stromal cells (AdMSCs) were isolated from young healthy donors by digestion with collagenase A. Briefly; tissue sample was washed, homogenized with Ika Ultra Turrax Drive and digested with collagenase A during two hours and a half at 37°C. The digestion was stopped with expansion medium at lower temperature. After centrifugation, semi-solid phase was filtered with 100 µm cell strainer, mixed with the pellet of cells and washed. The cells were counted in a Neubauer camera and seeded at 1·2-2·0x10⁴cells/cm². After 24h, the medium was changed to wash away any non-adherent cells. When 80-90% of confluence is reached, AdMSCs were trypsinized and reseeded in a density of 5x10³ cells/cm² for optimal proliferation. MSCs were characterized by the following specific markers: CD13, CD29, CD31, CD34, CD45, CD73, CD90, HLA-II and CD105 and by differentiation potential after cell expansion.

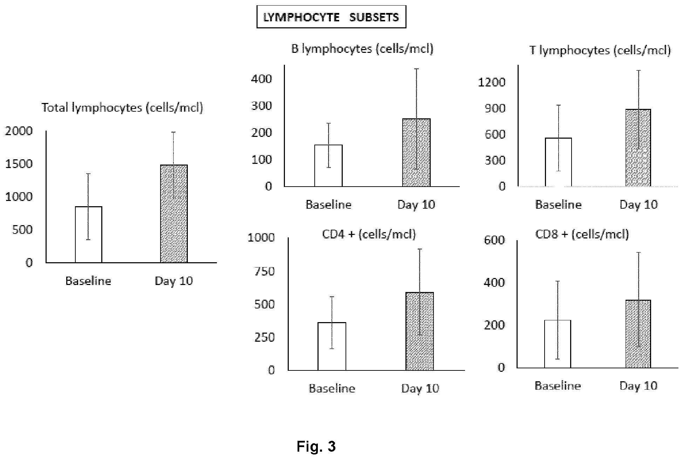

Figure 1 summarizes the results of 14 patients treated, 13 of them from Compassionate Uses of the BALMYS-19 Project (Bernat Soria Principal Investigator) in the Hospitals Gregorio Marañón and Fundacion Jimenez Diaz from Madrid, University Hospitals of Salamanca, Murcia and Alicante (BALMYS-19 Project) and Clínica Teknon-Institut de Terapia Regenerativa I Tisular (ITRT) in Barcelona (Compassionate Use after Confidential Agreement with the Inventor). All patients were treated with steroids, prophylactic antibiotics and low-molecular weight heparin, whilst eleven of them had been treated with lopinavir/ritonavir, hydroxychloroquine and tocilizumab before administration of the New Pharmaceutical Product. Important to note there were no adverse events related to cell therapy. In the Grade 5 to 7, seven patients were extubated and discharged from ICU while four patients remained intubated (two with an improvement in their ventilatory and radiological parameters and two in stable condition). Two patients died (one due to massive gastrointestinal bleeding unrelated to this therapy). A decrease in inflammatory parameters (Figure 2, reduction in C-reactive protein, IL-6, ferritin, LDH and D-Dimer) as well as an increase in lymphocytes, particularly in those patients with clinical improvement was observed after treatment (see Figure 2). In one Grade 4 patient all the parameters ameliorate and, very important, no pulmonary sequelae were observed 20 days after discharge.
Quite relevant is the observation that this ATMP do not inhibit the immune response (Figure 3). In contrast, total number of lymphocytes increase and also the lymphocyte subsets (B, T, NK, CD4, CD8) either increase or stay at similar figures. It may be concluded that the treatment with intravenous administration of the New Pharmaceutical Products in severe COVID-19 pneumonia under mechanical ventilation is safe and it is associated with clinical and biological improvement, whilst early treatment (Grade 3-4) may benefit all the parameters and discharge the patients with no sequelae.

### EXAMPLE 2: Administration and Biodistribution of ATMPs

A very relevant contribution of this invention is the design and practical demonstration of an administration protocol to infuse the cells that will concentrate and stay in the pulmonary tissues without producing an increase in the pulmonary artery pressure and other directly related adverse reactions observed in the case of pulmonary sepsis.

### Administration of the New Pharmaceutical Product to COVID-19 patients

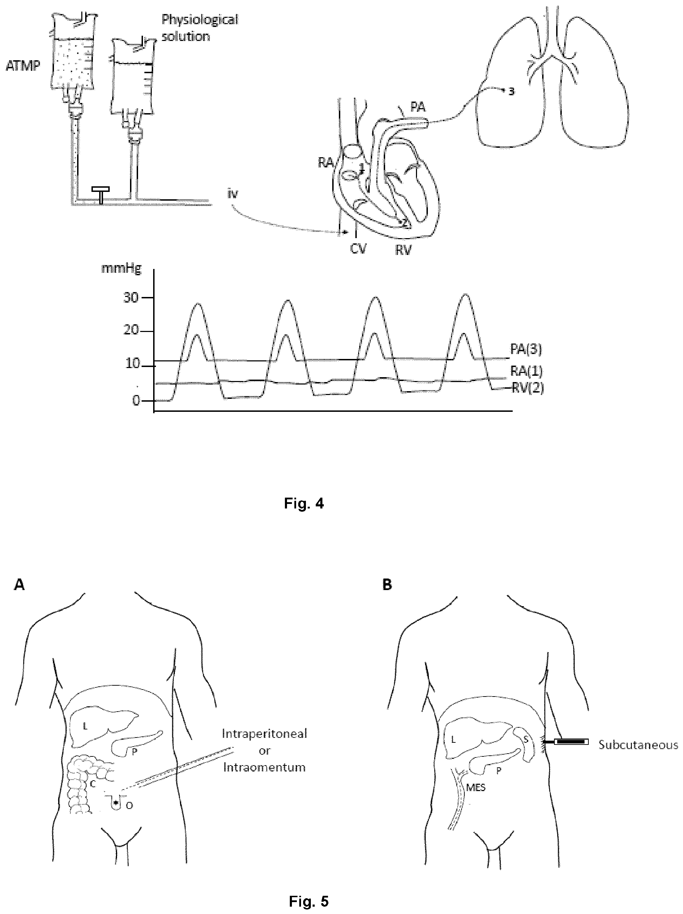

Strict Protocol for the Administration of the Cellular Medicament in COVID-19 (see Figure 4). The protocol consist in: a) Intravenous administration in one or more doses pf 0,5 to 1,5 million cells/kg per dose; b) very important: since cells are living organisms cannot be applied as usually done with molecular medicaments. Once they are thawed and washed several times with physiological solution to be used as excipient of the suspension not to form cellular aggregates; c) Gently and smoothly administered by gravity at a rate of 1-3 mL/min; d) Since most of cells are to be retained by the lungs, in order to monitor pulmonary artery pressure and Swan-Ganz catheter or similar is located in the pulmonary artery; e) Infusion of one dose of 70 to 100 million cells last usually 45 to 60 min.

### Combinatorial Therapies:

Similar ATMPs have shown to be safe in hundreds of Clinical Trials and we know by preclinical studies that lungs act as a filter retaining most of the cells intravenously injected. Since these patients suffer a prothrombotic state we suggest the concomitant use of low molecula weight heparin and/or defibrotide, a drug used in sinusoidal obstructive syndrome (SOS) after hematopoietic stem cell transplantation. Defibrotide is a mixture of single stranded oligonucleotide aptamers with a multitarget pharmacology limiting endothelial cell activation. Given its antithrombotic, anti-TNFa, antiatherosclerotic, etc. effects, the US Food and Drug Administration (FDA) approved its use in SOS. We suggest that it will be consistent to block both the cytokine storm and to prevent pulmonary thromboembolism.

### Administration of the New Pharmaceutical Product in other diseases.

Figure 5 describes different administration routes. For example, it shows the that intraperitoneal and intraomentum laparoscopic administration strategies used for NAFLD, liver fibrosis, autoimmune hepatitis, inflammatory bowel disease, Crohn's disease, ulcerative colitis, type 1 and type 2 diabetes and others. Under these conditions, preclinical data shows the displacement from M1 (pro-inflammatory) to M2 (anti-inflammatory) macrophages, increase of Treg, increase of anti-inflammatory cytokines (11-10) and decrease of pro-inflammatory cytokines.

### EXAMPLE 3: Obtention, culture expansion and characterization of Cellular Medicaments in a xeno-free and human component free media

Commonly used medium formulations for MSC cultivation are based on the addition of fetal bovine serum (FBS) as a source of growth factors and cytokines. FBS-supplemented medium is produced easily, but has substantial disadvantages when used for the *ex vivo* expansion of cells for clinical application. First, contamination with animal pathogens including specific viruses (such as SARS-CoV-2), mycoplasma, or prions cannot be fully excluded. Second, immune reactions to FBS proteins have been reported in patients receiving cell therapy. Therefore, serum-free media formulations or supplements devoid of xenogenic substances, such as human platelet lysate (HPL), are being employed to substitute for FBS in clinical applications, but this do not exclude human-to-human contamination. Even more, EV obtention using of MSC is enhanced in HPL compared with FBS. However, the high fibrinogen content present in HPL primes the formation of fibrin gels when added to the calcium-containing media. To prevent gel formation, porcine heparin is usually added to HPL-based growth media, which, however, introduces xenogenic substances. Despite the addition of heparin, such media are characterized by a short shelf life, and fibrin persistently precipitates during storage and cell culture, leading to cell compaction and uneven cell spreading. To circumvent these shortcomings, we have developed a method to culture and expand mesenchymal stromal cells in a chemically defined fetal bovine serum free and human platelet lysate free media.

A new culture media containing 3-10 mM glucose, 3-10 mM lithium chloride, 2-10 micromolar nitric oxide donor, 50-200 nM hydrocortisone, recombinant human growth factors and physiological concentration of nutrients. This new media reported in this invention avoids animal-to-human and human-to-human contamination and also precludes prothrombotic effects of HPL containing media. This is very relevant given the coagulation problems fo COVID patients. Figure 6 shows that the phenotype of the active principle is maintained after 28 days of growth in this media (Figure 6 A) and that the doubling time after 15 days is kept at low value (around 50 h). The former allowing massive production of cells and their derivatives.

### Cell proliferation

5-bromo-2-deoxyuridine (BrdU) incorporation into DNA was studied with the BrdU "Cell Proliferation Elisa BrdU colorimetric" (Roche). 300-500 cells were seeded by Cell Sorter (BD FACSAria) into 96-well plates on a Matrigel® layer, preparing 3-6 replicates per experimental condition. Biefly, cells were exposed to BrdU (1:100) 16 h before the end of exposure to hypoxia or DETA/ NO. Subsequently, cells were fixed and exposed anti-BrdU antibody (1: 100) for 2 h at RT. In order to allow the antibody to bind to the BrdU incorporated into the DNA, cells must be pre-fixed, permeabilized and DNA denatured, through Kit fixing solution (Fixdenat, Roche) for 30 minutes at RT. To remove the excess antibody, 3 washes with 200 µl of wash solution included in the kit were performed. Cells were then incubated with the substrate solution for 5-30 minutes at RT to finally stop the reaction with H₂SO₄. Finally, absorbance at 450 nm was measured which is proportional to the degree of cell proliferation.

### EXAMPLE 4: Safety of the new Pharmaceutical Product- avoiding prothrombotic effect of tentative ATMP

Inflammatory disease usually associated with coagulation problems, in type 2 diabetes there is a systemic inflammation in different tissues. This is there the reason why autologous mesenchymal stromal cells of type 2 diabetes patients may be prothrombotic instead of fibrinolytic [22]

### Coagulation problems in COVID19 and other inflammatory diseases

Excessive cytokine release and activation of coagulation appear to be the key drivers of COVID-19 pneumonia and associated mortality [23,24]. Approximately half of critical care COVID-19 patients present relevant thrombotic complications with pulmonary thromboembolism as a determinant factor (Table.. of High thrombotic Risk and Very High thrombotic risk). In contrast with SARS and MERS (Middle East Respiratory Disease) syndromes, SARS-CoV-2 produces much higher thrombotic and thromboembolic events such as: venous thromboembolism and arterial thrombosis. There is a high correlation between coagulation abnormalities and markers of thrombosis and hemostasis (platelet count, D-dimer, Prothrombin time, troponin) and clinical outcomes. Given the observations and the fact that mesenchymal stromal cells may be prothrombotic [22] it is compulsory to include testing the prothrombotic factors in the Quality Control of the ATMP [25]

### EXAMPLE 5: Treatment of Critical Ischemia of the Limbs (NOMA PROJECT)

Damage in vessels and capillaries may be a consequence of atherosclerosis, diabetes, metabolic syndrome, vasculitis, etc. In the case of COVID-19 in addition to coagulation problems there are cardiovascular problems, endothelial inflammation, etc. There is no enough time to see whether ourCOVID-19 patients recover the vascular homeostasis. In the meanwhile it may be useful to check the results suing umbilical cord mesenchymal stromal cells expanded in xeno-free and human-component-free chemically defined media. Patients were treated in the Toribio de Mendoza University (Peru) by Porf J Tejedo team under the direction of Porf Bernat Soria, Peru from September 2019 to February 2020

Peripheral Artery Disease (PAD) is the most prevalent manifestation of atherosclerosis. In people suffering from diabetes arterial narrowing at the lower limb level, causes an imbalance between supplementation and demand for oxygen, causing changes in the macro and microcirculation. It encompasses a clinical spectrum ranging from asymptomatic patients to the most severe form, Critical Limb Ischemia (CLI), which could result in amputation of the affected limb. Therefore, CLI is considered the final stage of PAD. The incidence of CLI could be between 500-1000 new cases per million every year in Western Europe and North America and this number is expected to grow due to a population with higher life expectancy and a progressive increase in diabetic people. These two factors favor the development of this more severe degree of PAD. It has been described that either mononuclear cells from the bone marrow or mesenchymal stromal cells from different origins may benefit vessels creation.

We have shown that "off-the-self" umbilical cord mesenchymal stromal cells obtained and cultured in a xeno-free and human component free chemically defined media administered intramuscularly at dose of 1 to 5 million cells / kg may induce vasculogenesis and angiogenesis. Given the vasculitis and endothelial inflammation and damage present in COVID-19 patients we do expect that these cells will ameliorate the condition of the cardiovascular problems in COVID-19 patients.

### EXAMPLE 6: Next generation of Advanced Therapy Medicinal Products-Obtaining Extracellular Vesicles (EVs) from Mesenchymal Stromal Cells in a xeno-free and human components free media

It has been postulated that MSCs exert different paracrine actions, including angiogenesis and immunoregulation through secreted factors. Some recent data suggest that the provision of viable MSCs to damaged tissues is not necessary to exert the therapeutic effects of Mesenchymal Stromal Cells [26]. Recently, the extracellular vesicles (EV) released by these cells are being explored as next-generation therapeutic candidates [27]. These biological products recapitulate in some aspects the therapeutic benefits exerted by the parental cells. Among the advantages of the use of EV is easier handling as inert biological products and their small size. In fact, the cell size of most adult stem cells limits the maximum dose that can be administered to avoid complications and the use of EVs released by these cells could overcome this problem. The clear disadvantage, solved with these inventions, is that mesenchymal stromal cells have to be immortalized using viruses and will be considered a gene therapy.

The EVs are lipid bilayer vesicles of 30 to 150 nm released by a wide variety of eukaryotic cells, including adult stem cells. These vesicles contain characteristic surface receptors and are loaded mainly with proteins and microRNAs. Although the composition of the protein is shared with other types of cells, it seems that the miRNA payload is specific and is very involved in cell-to-cell communication. This composition of miRNA in particular has a major contribution in the regeneration potential of the EVs and intensive research in the field is intended to unravel the key miRNAs of this cocktail. In this context, we and others have described variations in the load of miRNA when genetic modifications are induced in the parental cells [28]. These vesicles are capable of inducing angiogenesis, proliferation of myocytes, antiapoptotic effect, immunoregulatory responses and reduction of oxidative stress in vitro and in animal models of cardiovascular diseases [29].

### REFERENCES

1. Medzhitov R. Origin and physiological roles of inflammation. Nature. 2008 Jul 24;454(7203):428-35
2. Calliope A Dendrou, Lars Fugger, Manuel A Friese (2015) Immunopathology of Multiple Sclerosis Nat Rev Immunol 15(9):545-58.
3. Tay MZ, Poh CM, Rénia L, MacAry PA, Ng LFP. The trinity of COVID-19: immunity, inflammation and intervention. Nature Reviews Immunology. Published online April 28, 2020. doi:10.1038/s41577-020-0311-8
4. Moore BJB, June CH. Cytokine release syndrome in severe COVID-19. Science (New York, NY). Published online April 17, 2020. doi:10.1126/science.abb8925
5. Shi Y, Wang Y, Shao C, et al. COVID-19 infection: the perspectives on immune responses. Cell Death & Differentiation. Published online 2020. doi:10.1038/s41418-020-0530-3
6. Mehta P, McAuley DF, Brown M, Sanchez E, Tattersall RS, Manson JJ. COVID-19: consider cytokine storm syndromes and immunosuppression. The Lancet. 2020;395(10229):1033-1034. doi:10.1016/S0140-6736(20)30628-0
7. McGonagle D, Sharif K, O'Regan A, Bridgewood C. Interleukin-6 use in COVID-19 pneumonia related macrophage activation syndrome. Autoimmunity Reviews. Published online April 2020:102537. doi:10.1016/j.autrev.2020.102537
8. Ciceri F, Beretta L, Scandroglio AM, et al. Microvascular COVID-19 lung vessels obstructive thromboinflammatory syndrome (MicroCLOTS): an atypical acute respiratory distress syndrome working hypothesis. Critical care and resuscitation: journal of the Australasian Academy of Critical Care Medicine. Published online April 15, 2020. Accessed April 25, 2020. http://www.ncbi.nlm.nih.gov/pubmed/32294809
9. Zhang L, Yan X, Fan Q, et al. D-dimer levels on admission to predict in-hospital mortality in patients with Covid-19. Journal of Thrombosis and Haemostasis. Published online April 19, 2020. doi:10.1111/jth.14859
10. Connors JM, Levy JH. COVID-19 and its implications for thrombosis and anticoagulation. Blood. Published online April 27, 2020. doi: 10.1182/blood.2020006000
11. Bhatraju PK, Ghassemieh BJ, Nichols M, et al. Covid-19 in Critically III Patients in the Seattle Region - Case Series. The New England journal of medicine. Published online 2020:1-11. doi:10.1056/NEJMoa2004500
12. Grasselli G, Zangrillo A, Zanella A, et al. Baseline Characteristics and Outcomes of 1591 Patients Infected With SARS-CoV-2 Admitted to ICUs of the Lombardy Region, Italy. JAMA. Published online April 6, 2020. doi:10.1001/jama.2020.5394
13. Wynants L, van Calster B, Bonten MMJ, et al. Prediction models for diagnosis and prognosis of covid-19 infection: systematic review and critical appraisal. BMJ (Clinical research ed). 2020;369:m1328. doi:10.1136/bmj.m1328
14. Ji D, Zhang D, Xu J, et al. Prediction for Progression Risk in Patients with COVID-19 Pneumonia: the CALL Score. Clinical infectious diseases: an official publication of the Infectious Diseases Society of America. Published online April 9, 2020. doi:10.1093/cid/ciaa414
15. Leng Z, Zhu R, Hou W, et al. Transplantation of ACE2- Mesenchymal Stem Cells Improves the Outcome of Patients with COVID-19 Pneumonia. Aging and disease. 2020;11(2):216-228. doi:10.14336/ad.2020.0228
16. García-Gómez I, Elvira G, Zapata AG, et al. Mesenchymal stem cells: biological properties and clinical applications. Expert Opin Biol Ther 2010; 10: 1453-1468.
17. Hashi CK, Zhu Y, Yang GY, Young WL, Hsiao BS, Wang K, Chu B, Antithrombogenic property of bone marrow mesenchymal stem cells in nanofibrous vascular grafts. Proc Natl Acad Sci U S A. 2007 Jul 17;104(29):11915-20. Epub 2007 Jul 5.
18. Prockop DJ, Oh JYMesenchymal stem/stromal cells (MSCs): role as guardians of inflammation. Mol Ther. 2012 Jan;20(1):14-20. doi: 10.1038/mt.2011.211. Epub 2011 Oct
19. Adutler-Lieber, S., et al., Human macrophage regulation via interaction with cardiac adipose tissue-derived mesenchymal stromal cells. J Cardiovasc Pharmacol Ther, 2013. 18(1): p. 78-86.
20. Le Blanc, K. and D. Mougiakakos, Multipotent mesenchymal stromal cells and the innate immune system. Nat Rev Immunol, 2012. 12(5): p. 383-96.
21. Herreros MD, Garcia-Arranz M, Guadalajara H, De-La-Quintana P, Garcia-Olmo D; FATT Collaborative Group. Autologous expanded adipose-derived stem cells for the treatment of complex cryptoglandular perianal fistulas: a phase III randomized clinical trial (FATT 1: fistula Advanced Therapy Trial 1) and long-term evaluation. Dis Colon Rectum 2012; 55:762-772.
22. Acosta L, Hmadcha A, Escacena N, et al. Adipose mesenchymal stromal cells isolated from type 2 diabetic patients display reduced fibrinolytic activity. Diabetes 2013; 62: 4266-4269.
23. Vaninov N In the eye of the COVID-19 cytokine storm NNat Rev Immunol. 2020 May;20(5):277. doi: 10.1038/s41577-020-0305-6.
24. Helms J, Tacquard C, Severac F, Leonard-Lorant I, Ohana M, Delabranche X, Merdji H, Clere-Jehl R, Schenck M, Fagot Gandet F, Fafi-Kremer S, Castelain V, Schneider F, Grunebaum L, Angles-Cano E, Sattler L, Mertes PM, Meziani F CRICS TRIGGERSEP Group (Clinical Research in Intensive Care and Sepsis Trial Group for Global Evaluation and Research in Sepsis).High risk of thrombosis in patients with severe SARS-CoV-2 infection: a multicenter prospective cohort study. Intensive Care Med. 2020 May 4. doi: 10.1007/s00134-020-06062-x. [Epub ahead of print
25. Soria B et al METHOD FOR PREDICTING TREATMENT RESPONSE AND TEST FOR SAFE USE OF MESENCHYMAL STEM CELLS ON INFLAMMATORY DISEASES. PCT/EP2014/066600
26. Du R-H, Liang L-R, Yang C-Q, et al. Predictors of Mortality for Patients with COVID-19 Pneumonia Caused by SARS-CoV-2: A Prospective Cohort Study. The European respiratory journal. Published online April 8, 2020. doi:10.1183/13993003.00524-2020
27. Abu-Raya B. Predictors of refractory Coronavirus disease (COVID-19) pneumonia. Clinical infectious diseases: an official publication of the Infectious Diseases Society of America. Published online April 9, 2020. doi:10.1093/cid/ciaa409
28. Prockop, D.J., et al., Evolving paradigms for repair of tissues by adult stem/progenitor cells (MSCs). J Cell Mol Med, 2010. 14(9): p. 2190-9.
29. Wang, J., C. Zhao, and J. Xiao, Exosomes in Cardiovascular Diseases and Treatment: Exper. and Clinical Aspects. J Cardiovasc Transl Res, 2019. 12(1): p. 1-2.

## Claims

1. A pharmaceutical product consisting in a suspension of mesenchymal stromal cells (active principle) tested previously for prothrombotic factors, at a dose of 0.5 to 1,5 million of cells/kg in administered intravenously at a rate of 1-3 mL/min by a qualified, accredited and registered person with a valid medical degree. During the process pulmonary artery pressure or the portal pressure has to be controlled and kept under the physiological range.

2. A pharmaceutical product consisting in a suspension of mesenchymal stromal cells (active principle) tested previously for prothrombotic factors, at a dose of 1 to 5 million cells/kg in administered either intramuscularly by qualified, accredited and registered healthcare person.

3. A pharmaceutical product consisting in a suspension of mesenchymal stromal cells (active principle) tested previously for prothrombotic factors, at a dose of 1 to 10 million cells/kg in administered intraperitoneally by qualified, accredited and registered person with a valid medical degree. During the process intraperitoneal pressure has to be controlled and kept under physiological range.

4. A pharmaceutical product consisting in a suspension of mesenchymal stromal cells (active principle) tested previously for prothrombotic factors, at a dose of 1 to 10 million cells/kg in administered intraarterially close to the target tissue

5. A pharmaceutical product consisting in a suspension of mesenchymal stromal cells (active principle) tested previously for prothrombotic factors, at a dose of 1 to 10 million cells/kg in administered subcutaneously close to the target tissue

6. A pharmaceutical product consisting in a suspension of mesenchymal stromal cells (active principle) tested previously for prothrombotic factors, at a dose of 1 to 10 million cells/kg in administered though the nasal olfactory mucose.

7. A pharmaceutical product consisting in a suspension of extracellular vesicles obtained from mesenchymal stromal cells tested previously for low prothrombotic factors

8. Claims 1 to 7 when mesenchymal stromal cells (active principle) are cultured in a media free of products of animal origin, for example fetal bovine serum.

9. Claims 1 to 7 when mesenchymal stromal cells (active principle) are cultured under GMP conditions in a system that contains glucose 5 to 7 mM, lithium chloride 3-10 mM, nitric oxide donor 2 to 10 uM, hydrocortisone 50 to 200 nM, nutrients and recombinant growth factors.

10. Claims 1 to 7 when mesenchymal stromal cells (active principle) are cultured in a media free of products of animal origin, for example human platelet lysate.

11. Claims 1 to 7 when the mesenchymal stromal cells (active principle), are obtained from adipose tissue

12. Claims 1 to 7 when the mesenchymal stromal cells (active principle), are obtained from the bone marrow

13. Claims 1 to 7 when the mesenchymal stromal cells (active principle), are obtained from the dental pulp

14. Claims 1 to 7 when the mesenchymal stromal cells (active principle), are obtained from the umbilical cord.

15. Claims 1 to 7 when the mesenchymal stromal cells (active principle), are used immediately after thawing

16. Claims 1 to 7 when the mesenchymal stromal cells (active principle), are used after refreshing for 3 to 5 days

17. Claims 1 to 7 when the pharmaceutical product is an aerosol

18. Claims 1 to 7 when the pharmaceutical product is a cream.

19. Claims 1 to 7 when mesenchymal stromal cells are administered in combination with defibrotide, steroids, anti-IL6, anti-IL-1, abut TNF alfa, low molecular weight heparin and other anticoagulants.

20. Claims 1 to 7 when the pharmaceutical product is administered intrarterially for autoimmune and inflammatory disease

21. Claims 1 to 7 when the pharmaceutical product is administered intraperitoneally for autoimmune and inflammatory disease ,such as autoimmune hepatitis, tipe 1 and type 2 diabetes, inflammatory bowel disease, Crohn's disease, ulcerative colitis and others

22. Claims 1 to 7 when the pharmaceutical product is administered intrarticularly or in the core of the intervertebral disc for rheumatoid arthritis, osteoarthritis, backpain and others.

23. Claims 1 to 7 when the pharmaceutical product is administered locally to repair tendon lesions.
